# FASCICULE DE BREVET EUROPEEN

(11) **EP 1 686 978 B1**
(45) Date de publication et mention de la délivrance du brevet: **13.08.2008**
(21) Numéro de dépôt: 04805440.7
(22) Date de dépôt: 10.11.2004
(51) Int. Cl.: A61K 31/165, A61P 17/00

(54) **UTILISATION DE L'IDROCILAMIDE POUR LA PREPARATION D'UNE COMPOSITION PHARMACEUTIQUE POUR LE TRAITEMENT DE LA ROSACEE**
VERWENDUNG VON IDROCILAMID ZUR HERSTELLUNG EINER PHARMAZEUTISCHEN ZUSAMMENSETZUNG ZUR BEHANDLUNG VON ROSACEA
USE OF IDROCILAMIDE FOR THE PREPARATION OF A PHARMACEUTICAL COMPOSITION FOR THE TREATMENTOF ROSACEA

(30) Priorité: 21.11.2003 FR 0313666
(43) Date de publication de la demande: 09.08.2006
(73) Titulaire: GALDERMA RESEARCH & DEVELOPMENT, 06410 Biot (FR)
(72) Inventeur: DOLFI, Fabrizio, F-06560 Valbonne (FR); PILGRIM, William Robert, F-06560 Valbonne (FR)
(74) Mandataire: Bernstein, Claire Jacqueline
(86) Numéro de dépôt international: PCT/FR2004/002899
(87) Numéro de publication internationale: WO 2005/060950

(56) Documents cités:
- WO-A-02/074290
- US-A- 3 659 014
- BANNWARTH B ET AL: "[Tissue and systemic diffusion of idrocilamide after cutaneous administration]" décembre 1993 (1993-12), REVUE DU RHUMATISME (ED. FRANCAISE : 1993) DEC 1993, VOL. 60, NR. 12, PAGE(S) 932 - 936 , XP001193739 le document en entier
- DATABASE BIOSIS [Online] BIOSCIENCES INFORMATION SERVICE, PHILADELPHIA, PA, US; 1990, XU J-F ET AL: "STUDY OF PHARMACOLOGY AND TOXICOLOGY OF IDROCILAMIDE" XP002284400 Database accession no. PREV199191034330 & ZHONGGUO YIYAO GONGYE ZAZHI, vol. 21, no. 2, 1990, pages 70-72, ISSN: 1001-8255
- LOUIS DUBERTRET: "Thérapeutique Dermatologique" 1991, MEDICINE-SCIENCES FLAMMATION , PARIS * page 529 - page 530 *

## Description

La présente invention concerne le domaine du traitement de la rosacée. L'invention vise à fournir de nouvelles compositions pharmaceutiques, plus particulièrement dermatologiques utiles pour le traitement du premier ou du deuxième stade de la rosacée et comprenant à titre d'agent actif l'idrocilamide.

La rosacée est une dermatose inflammatoire commune chronique et progressive liée à une instabilité vasculaire. Elle affecte principalement la partie centrale du visage et se caractérise par le rougissement du visage ou les bouffées de chaleur, l'érythème facial, les papules, les pustules, et la télangiectasie. Dans les cas graves, particulièrement chez l'homme, le tissu mou du nez peut enfler et produire un gonflement bulbeux appelé rhinophyma.

La rosacée survient généralement entre l'âge de 25 et 70 ans, et elle est beaucoup plus commune chez les gens au teint clair. Elle touche plus particulièrement les femmes, bien que cette affection soit généralement plus sévère chez l'homme. La rosacée est chronique et persiste des années avec des périodes d'exacerbation et de rémission.

La rosacée a originellement été appelée "acné rosacée" parce que ses papules (légères surélévations de la peau) et ses pustules inflammatoires (croûtes de pus) ressemblent beaucoup à celles de l'acné vulgaire. Contrairement à l'acné vulgaire, dont l'étiologie est fondée à la fois sur une kératinisation anormale, une augmentation de la production de sébum et une inflammation bactérienne, l'inflammation de la rosacée est de nature vasculaire et mal comprise. Il résulte de cette anomalie vasculaire faciale un oedème permanent du derme qui pourrait accompagner une colonisation accrue par *Demodex folliculorum,* acarien qu'on trouve habituellement dans les follicules du visage. Ce parasite pourrait déclencher des phénomènes inflammatoires se traduisant par des papules et des pustules.

La pathogenèse de la rosacée est mal connue. De nombreux facteurs peuvent être impliqués sans forcément induire cette affection. Ce sont par exemple des facteurs psychologiques, des troubles gastro-intestinaux, des facteurs environnementaux (exposition au soleil, température, humidité) et émotionnels (stress), alimentaires (alcool, épices), hormonaux, vasculaires, voire une infection par *Helicobacter pilori.*

La rosacée évolue en 4 stades, mais le passage par tous les stades n'est pas obligatoire:
- stade 1 des bouffées vasomotrices (vers 20 ans). Les patients ont des poussées soudaines de rougeur paroxystique du visage et du cou, avec sensation de chaleur, mais sans signes systémiques. Après les crises, la peau du visage redevient normale. Ces «flushes » sont déclenchés par les changements de température (entraînant parfois une thermophobie), l'absorption de boissons chaudes ou d'alcool.
- stade 2 érythémato-télangiectasique (vers 30 ans). Les zones malaires sont diffusément rouges. On y observe des capillaires dilatés constituant la classique couperose. A la différence du stade 1, la rougeur est permanente. Outre les joues, le menton et la partie médiane du front peuvent être touchés.
- stade 3 des papulo-pustules (vers 40 ans). Sur un fond d'érythème se développent des papules et des pustules de quelques millimètres de diamètre, sans comédons associés. Cette dermatose peut être très étendue, parfois à la partie glabre du cuir chevelu chez l'homme, mais respecte le pourtour de la bouche et des yeux. Les patients se plaignent d'une peau sensible, avec intolérance subjective à la plupart des topiques et des cosmétiques gras.
- stade 4 du rhinophyma (vers 50 ans ou plus tard). Cette phase tardive touche principalement les hommes, contrairement aux autres stades. Le nez est augmenté de volume, diffusément rouge et les orifices folliculaires sont dilatés. La peau s'épaissit progressivement.

Les formes mineures de la rosacée peuvent être traitées par des actifs tels que les anti-séborrhéiques et les anti-infectieux, par exemple le peroxyde de benzoyle, l'acide rétinoïque, le métronidazole (antiparasitaire). Quant aux formes les plus diffuses de l'affection, elles répondent bien à une antibiothérapie générale par les cyclines. Cependant, ces traitements présentent des effets secondaires désagréables pour le patient tels des phénomènes d'irritation ou d'intolérance.

De plus, en raison de l'aspect multi-factoriel de la rosacée, il existe de très nombreuses thérapies contre cette affection, mais on est toujours à la recherche d'un traitement efficace et sans risque pour le patient.

La Demanderesse a maintenant mis en évidence les propriétés intéressantes d'un composé appartenant à la famille des anti-inflammatoires non stéroïdiens (les AINS), l'idrocilamide, pour le traitement du premier ou du deuxième stade de la rosacée.

Les AINS sont classés en fonction de leur structure chimique :
- dérivés d'acide salicylique (par exemple, aspirine, sulfasalazine, salicylate de sodium, salsalate, diflunisal, olsalazine) ;
- dérivés para-aminophénol (par exemple acetaminophen) ;
- indole et acides acétiques d'indole (par exemple indomethacin, sulindac, etodolac) ;
- acides acétiques aryl (par exemple, tolmetin, diclofenac, ketorolac) ;
- acides arylpropioniques (par exemple ibuprofen, naproxen, ketopropfen, idrocilamide, fenoprofen, oxaprozin) ;
- acides anthraniniques (fenamates) (par exemple acide mefanamique, acide meclofenamique) ;
- acides énoliques (par exemple oxicames (piroxicam, tenoxicam), pyrazolidiones (phénylbutazone, oxyphenthratazone)) ;
- alkanones (par exemple nabumetone).

Les AINS sont des composés anti-inflammatoires connus dans l'art antérieur pour leurs propriétés analgésique et anti-pyrétique. L'idrocilamide, ou N-(2-hydroxyethyl)cinnamide, est notamment commercialisé par la société Merck, dans la composition pharmaceutique Srilane pour ses propriétés de myorelaxant. L'idrocilamide répond à la formule suivante :

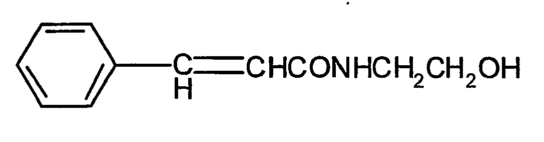

Par ailleurs, la demande de brevet EP 0270316 décrit l'utilisation de AINS dans des compositions topiques, en association avec l'imidazole substitué en 1, pour le traitement de l'acné. La demande de brevet internationale WO 02/074290 divulgue l'utilisation de certains AINS dans des préparations pharmaceutiques destinées à traiter la rosacée.

Cependant, il n'avait jamais été proposé d'utiliser l'idroicilamide pour le traitement de la rosacée. Dans le cadre de la présente invention, il a maintenant été trouvé que l'idrocilamide présente des propriétés particulièrement intéressantes dans le traitement de la rosacée, comme notamment une efficacité accrue en particulier chez les sujets à peau claire ou sensible, un amoindrissement considérable des effets secondaires, une efficacité probable au premier et au deuxième stade de la rosacée et une limitation des phénomènes de recrudescence.

Comme indiqué précédemment, l'invention vise à offrir une nouvelle composition pharmaceutique, préférentiellement dermatologique, destinée au traitement du premier ou du deuxième stade de la rosacée consistant à administrer par voie topique à un individu atteint de cette affection une quantité efficace d'idrocilamide.

En conséquence l'invention se rapporte plus particulièrement à l'utilisation de l'idrocilamide pour la préparation d'une composition pharmaceutique, plus particulièrement dermatologique, pour administration topique sur la peau, destinée au traitement du premier ou du deuxième stade de la rosacée.

Par traitement de la rosacée, on entend selon la présente invention, le traitement et/ou la prévention de la rosacée, au premier et/ou deuxième stade.

Suivant un premier mode de mise en oeuvre de l'invention, la composition est destinée au traitement du premier stade de la rosacée.

Suivant un deuxième mode de mise en oeuvre de l'invention, la composition est destinée au traitement du deuxième stade de la rosacée.

Suivant un premier mode préférentiel de mise en oeuvre, la composition contient 0,0001 à 20 %, d'idrocilamide, et plus préférentiellement 0,001 à 10 %, d'idrocilamide (exprimé en pourcentage en poids).

Suivant un deuxième mode préférentiel de mise en oeuvre, la composition contient 0,1 à 5%, d'idrocilamide (exprimé en pourcentage en poids).

Suivant un troisième mode préférentiel de mise en oeuvre, la composition sous forme de crème contient de l'ordre de 5 % d'idrocilamide (exprimé en pourcentage en poids).

Avantageusement, les compositions de l'invention comprennent outre l'idrocilamide au moins un autre agent thérapeutique susceptible d'augmenter l'efficacité du traitement. A titre d'exemples de tels agents, on peut citer des antibiotiques, des antibactériens, des antiviraux, des antiparasitaires, des antifongiques, des anesthésiques, des analgésiques, des antiallergiques, des rétinoïdes, des anti-radicaux libres, des antiprurigineux, des kératolytiques, des antiséborrhiques, des anti-histaminiques, des sulfures, des produits immunosuppresseurs ou antiprolifératifs.

Suivant un mode préférentiel de mise en oeuvre, la composition de la présente invention contient également du métronidazole.

Par métronidazole, on entend notamment le 1-(2-hydroxyethyl)-2-methyl-5-nitroimidazole mais aussi ses analogues et dérivés, notamment solubles dans les excipients de formulation adaptés pour la forme galénique utilisée.

Les compositions de l'invention peuvent comprendre en outre tout additif usuellement utilisé dans le domaine pharmaceutique, ou dermatologique, compatible avec l'idrocilamide. On peut citer notamment des séquestrants, des antioxydants, des filtres solaires, des conservateurs, par exemple la DL-alpha-tocophérol, des charges, des électrolytes, des humectants, des colorants, de bases ou d'acides usuels, minéraux ou organiques, des parfums, des huiles essentielles, des actifs cosmétiques, des hydratants, des vitamines, des acides gras essentiels, des sphingolipides, des composés autobronzants tels que la DHA, des agents apaisants et protecteurs de la peau tels que l'allantoïne, des agents propénétrants, des gélifiants. Bien entendu l'homme du métier veillera à choisir ce ou ces éventuels composés complémentaires, et/ou leur quantité, de manière telle, que les propriétés avantageuses de la composition selon l'invention ne soient pas, ou substantiellement pas, altérées.

Ces additifs peuvent être présents dans la composition à raison de 0 à 20 % en poids par rapport au poids total de la composition.

On peut citer comme exemples d'agents séquestrants, l'acide éthylènediamine tétracétique (EDTA), ainsi que ses dérivés ou ses sels, la dihydroxyethylglycine, l'acide citrique, l'acide tartrique, ou leurs mélanges.

On peut citer comme exemples de conservateurs le chlorure de benzalkonium, le phénoxyéthanol, l'alcool benzylique, la diazolidinylurée, les parabens, ou leurs mélanges.

On peut citer comme exemples d'agents humectants, la glycérine et le sorbitol.

Les compositions de l'invention peuvent contenir un ou plusieurs agents propénétrants dans des concentrations préférentielles allant de 0 à 20 % et plus préférentiellement allant de 0,6 à 3 % en poids par rapport au poids total de la composition. Parmi les agents propénétrants, on utilise préférentiellement, sans que cette liste soit limitative, des composés tels que le propylène glycol, le dipropylène glycol, le propylène glycol dipélargonate, le lauroglycol, l'éthoxydiglycol.

Avantageusement, les compositions selon l'invention peuvent contenir également un ou plusieurs agents tensioactifs liquides mouillants dans des concentrations préférentielles allant de 0 à 10 % et plus préférentiellement allant de 0,1 à 2 %.

Parmi les agents mouillants, on utilise préférentiellement, des composés de la famille des Poloxamers et plus particulièrement le Poloxamer 124 et/ou le Poloxamer 182.

Les compositions de la présente invention peuvent se présenter sous toutes les formes galéniques normalement utilisées pour une application topique, notamment sous forme de solutions aqueuses, hydroalcooliques ou huileuses, de dispersions du type lotion ou sérum, de gels aqueux, anhydres ou lipophiles, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-liquide ou solide du type crème, gel ou pommade ou encore de micro émulsions, de micro capsules, de micro particules ou de dispersions vésiculaires de type ionique et/ou non ionique.

De préférence les crèmes peuvent être formulées à partir d'un mélange d'huile minérale, ou d'un mélange de cire d'abeille et d'eau qui s'émulsifie instantanément, dans lequel on additionne l'idrocilamide dissout dans une petite quantité d'huile telle que l'huile d'amande.

Les pommades peuvent être formulées en mélangeant une solution d'idrocilamide dans une huile telle que l'huile d'amande dans de la paraffine chauffée, puis en laissant refroidir le mélange.

A titre d'exemples de compositions selon l'invention, on peut citer celles comprenant une phase active contenant (exprimé en pourcentage en poids) :
- 0 à 90 %, préférentiellement 5 à 25 %, notamment 10 à 20%, d'eau;
- 0 à 10 %, préférentiellement 0 à 2 %, notamment 0 à 0,5 %, de tensioactif liquide mouillant ;
- 0 à 20 %, préférentiellement 0 à 10 %, notamment 2 à 5 %, de propénétrant ;
- 0,0001 à 20 %, préférentiellement 0,001 à 10%, d'idrocilamide;
et une phase aqueuse comprenant un gélifiant pH-indépendant, et de l'eau.

La phase aqueuse d'une composition selon l'invention se présentant sous la forme d'une émulsion peut comprendre de l'eau, une eau florale telle que l'eau de bleuet, ou une eau thermale ou minérale naturelle, par exemple choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avène ou l'eau d'Aix les Bains.

Ladite phase aqueuse peut être présente à une teneur comprise entre 10 et 90 % en poids par rapport au poids total de la composition, de préférence comprise entre 20 et 80 % en poids.

A titre d'exemptes on peut citer les gélifiants de la famille des polyacrylamides tels que le mélange Sodium acryloyldimethyltaurate copolymer / isohexadecane / polysorbate 80 vendu sous le nom Simulgel 600 par la société Seppic, le mélange polyacrylamide / isoparaffine C13-14 / laureth-7 comme, par exemple, celui vendu sous le nom de Sepigel 305 par la société Seppic, la famille des polymères acryliques couplés à des chaînes hydrophobes tel que le PEG-150/decyl/SMDI copolymer vendu sous le nom de Aculyn 44 (polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35 % en poids dans un mélange de propylèneglycol (39 %) et d'eau (26 %)), la famille des amidons modifiés tels que l'amidon de pomme de terre modifié vendu sous le nom de Structure Solanace ou bien leurs mélanges.

Les gélifiants préférés sont issus de la famille des polyacrylamides tel que le Simulgel 600 ou le Sepigel 305 ou leurs mélanges.

Le gélifiant tel que décrit ci-dessus peut être utilisé aux concentrations préférentielles allant de 0,1 à 15% et, plus préférentiellement, allant de 0,5 à 5 %.

Les gels peuvent être préparés de préférence en dispersant ou en dissolvant l'idrocilamide dans un rapport approprié, dans un gel de type carbomère, poloxamère ou cellulosique.

### EXEMPLE 1 - COMPOSITIONS

Dans cet exemple, diverses formulations concrètes à base des composés selon l'invention sont illustrées.

### VOIE TOPIQUE

(a) Onguent
   - Idrocilamide 0,020 g
   - Myristate d'isopropyle 81,700 g
   - Huile de vaseline fluide 9,100 g
   - Silice 9,180 g
(b) Onguent
   - Idrocilamide 0,300 g
   - Vaseline blanche codex qsp 100 g
(c) Crème Eau-dans-Huile non ionique
   - Idrocilamide 0,100 %
   - Mélange d'alcools de lanoline émulsifs, de cires et d'huiles 39,900 %
   - Parahydroxybenzoate de méthyle 0,075 %
   - Parahydroxybenzoate de propyle 0,075 %
   - Eau déminéralisée stérile qsp 100 %
(d) Lotion
   - Idrocilamide 0,100 %
   - Polyéthylène glycol (PEG 400) 69,900 %
   - Ethanol à 95% 30,000 %
(e) Onguent hydrophobe
   - Idrocilamide 0,300 %
   - Miristate d'isopropyle 36,400 %
   - Huile de silicone ("Rhodorsil 47 V 300" vendu par RHONE-POULENC) 36,400 %
   - Cire d'abeille 13,600 %
   - Huile de silicone ("Abil 300.000 cst" vendu par GOLDSCHMIDT) qsp 100 %

## Revendications

1. Utilisation de l'idrocilamide pour la préparation d'une composition pharmaceutique, plus particulièrement dermatologique pour application topique, destinée au traitement du premier ou du deuxième stade de la rosacée.

2. Utilisation selon la revendication 1, **caractérisée en ce que** la composition contient de l'ordre de 0,0001 à 20 % en poids, de préférence de 0,001 à 10 % en poids et plus préférentiellement de 0,1 à 5 % d'idrocilamide en poids.

3. Utilisation selon l'une quelconque des revendications 1 à 2, **caractérisée en ce que** ladite composition contient en outre un autre agent actif notamment choisi dans le groupe des antibiotiques, des antibactériens, des antiviraux, des antiparasitaires, des antifongiques, des anesthésiques, des analgésiques, des antiallergiques, des rétinoïdes, des anti-radicaux libres, des antiprurigineux, des kératolytiques, des antiséborrhiques, des anti-histaminiques, des sulfures, des produits immunosuppresseurs ou antiprolifératifs.

4. Utilisation selon l'une des revendications 1 à 3, **caractérisée en ce que** ladite composition contient en outre du métronidazole.

5. Utilisation selon l'une quelconque des revendications 1 à 4, **caractérisée en ce que** la composition contient un additif choisi dans le groupe des séquestrants, des antioxydants, des filtres solaires, des conservateurs, des charges, des électrolytes, des humectants, des colorants, de bases ou d'acides usuels, minéraux ou organiques, des parfums, des huiles essentielles, des actifs cosmétiques, des hydratants, des vitamines, des acides gras essentiels, des sphingolipides, des composés autobronzants, des agents apaisants et protecteurs de la peau, des agents propénétrants, des gélifiants ou un mélange de ceux-ci.

## Claims

1. Use of idrocilamide for the preparation of a pharmaceutical composition and more particularly a dermatological composition for topical application, for treating the first or second stage of rosacea.

2. Use according to Claim 1, **characterized in that** the composition contains from about 0.0001% to 20% by weight, preferably from 0.001% to 10% by weight and more preferentially from 0.1% to 5% by weight of idrocilamide.

3. Use according to either of Claims 1 and 2, **characterized in that** the said composition also contains another active agent chosen especially from the group of antibiotics, antibacterial agents, antiviral agents, antiparasitic agents, antifungal agents, anaesthetics, analgesics, antiallergic agents, retinoids, free-radical scavengers, antipruriginous agents, keratolytic agents, anti-seborrhoeic agents, antihistamines, sulfides, immunosuppressant products and antiproliferative agents.

4. Use according to one of Claims 1 to 3, **characterized in that** the said composition also contains metronidazole.

5. Use according to any one of Claims 1 to 4, **characterized in that** the composition contains an additive chosen from the group of sequestrants, antioxidants, sunscreens, preserving agents, fillers, electrolytes, humectants, dyes, common mineral or organic acids or bases, fragrances, essential oils, cosmetic active agents, moisturizers, vitamins, essential fatty acids, sphingolipids, self-tanning compounds, skin calmative and protective agents, pro-penetrating agents and gelling agents, or a mixture thereof.

## Patentansprüche

1. Verwendung von Idrocilamid für die Herstellung einer pharmazeutischen Zusammensetzung und insbesondere einer dermatologischen Zusammensetzung zur topischen Anwendung, die für die Behandlung des ersten oder des zweiten Stadiums von Rosacea vorgesehen ist.

2. Verwendung nach Anspruch 1, **dadurch gekennzeichnet, dass** die Zusammensetzung Idrocilamid in einer Menge in der Größenordnung von 0,0001 bis 20 Gew.-%, vorzugsweise 0,001 bis 10 Gew.-% und noch bevorzugter 0,1 bis 5 % enthält.

3. Verwendung nach einem der Ansprüche 1 bis 2, **dadurch gekennzeichnet, dass** die Zusammensetzung ferner einen Wirkstoff enthält, der insbesondere unter den Antibiotika, antibakteriellen Wirkstoffen, antiviralen Wirkstoffen, antiparasitären Wirkstoffen, antimykotischen Wirkstoffen, Anesthetika, Analgetika, antiallergischen Wirkstoffen, Retinoiden, Radikalfängern für freie Radikale, Antipruriginosa, Keratolytika, Antiseborrhoika, Antihistaminika, Sulfiden, Immunsuppressiva oder antiproliferativen Produkten ausgewählt ist.

4. Verwendung nach einem der Ansprüche 1 bis 3, **dadurch gekennzeichnet, dass** die Zusammensetzung ferner Metronidazol enthält.

5. Verwendung nach einem der Ansprüche 1 bis 4, **dadurch gekennzeichnet, dass** die Zusammensetzung einen Zusatzstoff enthält, der unter den Maskierungsmitteln, Antioxidantien, Sonnenschutzfiltern, Konservierungsmitteln, Füllstoffen, Elektrolyten, Feuchthaltemitteln, Farbmitteln, üblichen organischen oder anorganischen Basen oder Säuren, Parfums, etherischen Ölen, kosmetischen Wirkstoffen, Hydratisierungsmitteln, Vitaminen, essentiellen Fettsäuren, Sphingolipiden, Selbstbräunungsmitteln, beruhigenden Stoffen und die Haut schützenden Stoffen, penetrationsfördernden Stoffen, Gelbildnern oder Gemischen dieser Verbindungen ausgewählt ist.
